# EUROPEAN PATENT SPECIFICATION

(11) **EP 2 425 250 B1**
(45) Date of publication and mention of the grant of the patent: **26.02.2014**
(21) Application number: 10716450.1
(22) Date of filing: 27.04.2010
(51) Int. Cl.: G01N 33/50, G01N 33/564

(54) **METHODS FOR MONITORING THE EFFICACY OF ANTI-IL-2R ANTIBODIES IN MULTIPLE SCLEROSIS PATIENTS**
VERFAHREN ZUR ÜBERWACHUNG DER EFFIZIENZ VON ANTI-IL-2R ANTIKÖRPERN IN MULTIPLE SKLEROSE PATIENTEN
PROCÉDÉS DE SURVEILLANCE DE L'EFFICACITÉ D'ANTICORPS ANTI-IL-2R CHEZ DES PATIENTS ATTEINTS DE SCLÉROSE EN PLAQUES

(30) Priority: 27.04.2009 US 173138 P
(43) Date of publication of application: 07.03.2012
(73) Proprietor: AbbVie Biotherapeutics Inc., Redwood City, CA 94063 (US)
(72) Inventor: SHERIDAN III, James Peter, Mountain View California 94040 (US)
(74) Representative: Roques, Sarah Elizabeth
(86) International application number: PCT/US2010/032572
(87) International publication number: WO 2010/126890

(56) References cited:
- WYNN DANIEL ET AL: "Daclizumab in active relapsing multiple sclerosis (CHOICE study): a phase 2, randomised, double-blind, placebo-controlled, add-on trial with interferon beta." April 2010 (2010-04), LANCET NEUROLOGY APR 2010 LNKD- PUBMED:20163990, VOL. 9, NR. 4, PAGE(S) 381 - 390 , XP026966728 ISSN: 1474-4465 abstract, p. 387, col. 1, line 5, col. 2, line 12, table 3, p. 388, fig. 5
- BIELEKOVA BIBIANA ET AL: "Effect of anti-CD25 antibody daclizumab in the inhibition of inflammation and stabilization of disease progression in multiple sclerosis." ARCHIVES OF NEUROLOGY APR 2009 LNKD- PUBMED:19364933, vol. 66, no. 4, 1 April 2009 (2009-04-01), pages 483-489, XP009129266 ISSN: 1538-3687
- HEINRICH A ET AL: "Immunophenotypic patterns of T-cell activation in neuroinflammatory diseases." ACTA NEUROLOGICA SCANDINAVICA APR 2006 LNKD- PUBMED:16542164, vol. 113, no. 4, April 2006 (2006-04), pages 248-255, XP002587544 ISSN: 0001-6314
- BATTISTINI LUCA ET AL: "CD8+ T cells from patients with acute multiple sclerosis display selective increase of adhesiveness in brain venules: a critical role for P-selectin glycoprotein ligand-1." BLOOD 15 JUN 2003 LNKD- PUBMED:12595306, vol. 101, no. 12, 15 June 2003 (2003-06-15), pages 4775-4782, XP002291380 ISSN: 0006-4971

### SEQUENCE LISTING

<110> Facet Biotech corporation
<120> METHODS FOR MONITORING THE EFFICACY OF ANTI-IL-2R ANTIBODIES IN MULTIPLE SCLEROSIS PATIENTS
<130> 222 WO 01
<150> US 61/173,138
   <151> 2009-04-27
<160> 2
<170> PatentIn version 3.5
<210> 1
   <211> 116
   <212> PRT
   <213> Artificial
<220>
   <223> Synthetic construct. "variable region of the PDL humanized anti-Tac antibody heavy chain."
<400> 1
   Gln Val Gln Leu Val Gln Ser Gly Ala Glu Val Lys Lys Pro Gly Ser
   1               5                   10                  15
   Ser Val Lys Val Ser Cys Lys Ala Ser Gly Tyr Thr Phe Thr Ser Tyr
               20                  25                  30
   Arg Met His Trp Val Arg Gln Ala Pro Gly Gln Gly Leu Glu Trp Ile
           35                  40                  45
   Gly Tyr Ile Asn Pro Ser Thr Gly Tyr Thr Glu Tyr Asn Gln Lys Phe
       50                  55                  60
   Lys Asp Lys Ala Thr Ile Thr Ala Asp Glu Ser Thr Asn Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Gly Gly Gly Val Phe Asp Tyr Trp Gly Gln Gly Thr Leu Val
               100                 105                 110
   Thr Val Ser Ser
           115
<210> 2
   <211> 106
   <212> PRT
   <213> Artificial
<220>
   <223> Synthetic construct. "Variable region of the PDL humanized anti-Tac antibody light chain."
<400> 2
   Asp Ile Gln Met Thr Gln Ser Pro Ser Thr Leu Ser Ala Ser Val Gly
   1               5                   10                  15
   Asp Arg Val Thr Ile Thr Cys Ser Ala Ser Ser Ser Ile Ser Tyr Met
               20                  25                  30
   His Trp Tyr Gln Gln Lys Pro Gly Lys Ala Pro Lys Leu Leu Ile Tyr
           35                  40                  45
   Thr Thr Ser Asn Leu Ala Ser Gly Val Pro Ala Arg Phe Ser Gly Ser
       50                  55                  60
   Gly Ser Gly Thr Glu Phe Thr Leu Thr Ile Ser Ser Leu Gln Pro Asp
   65                  70                  75                  80
   Asp Phe Ala Thr Tyr Tyr Cys His Gln Arg Ser Thr Tyr Pro Leu Thr
                   85                  90                  95
   Phe Gly Gln Gly Thr Lys Val Glu Val Lys
               100                 105

## Description

### 3. BACKGROUND

The goal of multiple sclerosis (MS) treatment is to prevent permanent disabilities and delay disease progression. A shorter-term therapeutic goal focuses on reducing the relapse rate. IFN-beta is the most commonly used chronic maintenance agent for treating MS. Drawbacks to IFN-beta treatment include inadequate response in at least 30% of patients following initiation of IFN-beta therapy (Bertolotto, et al., 2002, J Neurosurg Psychiatry, 73(2):148-153; and Durelli, 2004, J Neurol., 251 Suppl4:IV13-IV24), and induction of anti-interferon antibodies in 7% to 42% of patients (Sorensen, 2003, Lancet, 362:1184-91). Although other agents are used to treat MS, including corticosteroids, glatiramer acetate, mitoxantrone, and natalizumab, these agents are only partially effective in managing clinical relapses, and some carry significant safety risks.

Because the agents currently being used to treat MS are not completely effective in managing the disease, it is desirable to identify and clinically validate markers that can be used to monitor the clinical response in an individual diagnosed with MS to a therapeutic agent as means for optimizing and/or changing the treatment regime for that individual.

### 4. SUMMARY

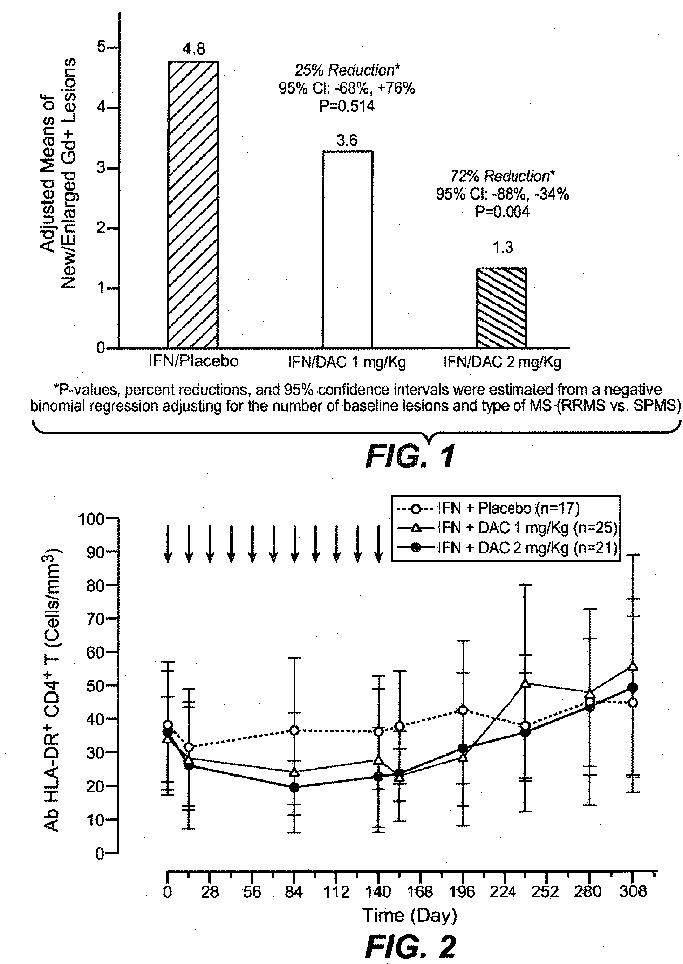

One agent that has shown promise for treating MS in clinical trials is daclizumab. Daclizumab was evaluated as a treatment for MS in a Phase 2, randomized, double-blinded, placebo-controlled, multi-center, dose-ranging study (the CHOICE study). At the end of the 24-week dosing period, compared to IFN-beta placebo, there was a 25% reduction in new or enlarged gadolinium contrast enhancing lesions (Gd-CEL) as detected by magnetic resonance imaging (MRI) in the daclizumab 1 mg/kg group and a 72% reduction in the daclizumab 2 mg/kg group (FIG. 1). Both daclizumab regimes were associated with an approximate 35% reduction in annualized relapse rate at 24 weeks (Montalban, X. et al., Multiple Sclerosis, 13: S 18-S 18 Suppl. 2 OCT 2007; and, Kaufman, M.D., et.a., Neurology, 70 (11): A220-A220 Suppl. 1 MAR 11 2008).

Patient blood samples were collected throughout the CHOICE study and analyzed to determine levels of immune cell subsets and associated activation markers prior to, during, and after treatment with daclizumab. Rapid, reversible, reductions in HLA-DR⁺CD4⁺ T cells, a subset of T cells, were observed during the daclizumab treatment phase for the 1 mg/kg and 2 mg/kg dosing groups. The exposure-dependent reductions in activated T cell numbers were consistent with the exposure-dependent reductions in new or enlarged Gd-CEL and reductions of annualized relapse rate.

These results indicate that changes in HLA-DR⁺CD4⁺ T cell counts can be used as a biomarker of clinical response to daclizumab in a patient diagnosed with MS. Accordingly, the methods described herein disclose the use of HLA-DR⁺CD4⁺ T cell counts to monitor the efficacy of daclizumab in a patient diagnosed with MS. In some embodiments, the method comprises determining the level of HLA-DR⁺CD4⁺ T cells in a patient diagnosed with MS following exposure to daclizumab, wherein a decrease in the level of HLA-DR⁺CD4⁺ T cells indicates that daclizumab is effective in ameliorating at least one symptom of MS in the treated patient. Biological samples from the treated patient can be collected at one or more times prior to, during, and/or after treatment with daclizumab.

Symptoms of MS that can be stabilized or improved using the methods described herein include, but are not limited to, reducing the relapse rate, stabilising or reducing the rate of disability progression as measured by standard scores such as the Expanded Disability Status Scale (EDSS) score, decreasing the number of new or enlarged Gd-CEL, and/or decreasing the number of new or enlarged T2 MRI lesions. The subject being treated can have relapsing forms of MS, including relapsing/remitting MS, secondary progressive MS, progressive relapsing MS, or worsening relapsing MS. In addition to daclizumab, other IL-2R antibodies, such as monoclonal antibodies, chimeric antibodies, humanized antibodies, or fully human antibodies that specifically bind to the alpha or p55 (Tac) chain of the IL-2 receptor can be used in the methods described herein.

It is to be understood that both the foregoing general description and the following detailed description are exemplary and explanatory only and are not restrictive of the compositions and methods described herein. In this application, the use of the singular includes the plural unless specifically state otherwise. Also, the use of "or" means "and/or" unless state otherwise. Similarly, "comprise," "comprises," "comprising," "include," "includes" and "including" are not intended to be limiting.

### 5. BRIEF DESCRIPTION OF THE FIGURES

FIG. 1 is a bar graph depicting data demonstrating the reduction in gadolinium contrast enhancing lesions at the end of a 24 week dosing period with daclizumab;
FIG. 2 is a line graph depicting data demonstrating rapid, reversible, reductions in HLA-DR⁺CD4⁺ activated T cells during daclizumab treatment phase; and
FIG. 3 is a line graph depicting data demonstrating the relationship between daclizumab steady state area under the curve (AUC) and decreases in HLA-DR⁺CD4⁺ activated T cell counts wherein a reduction in HLA-DR⁺CD4⁺ cells is represented as a positive number on the y-axis.

### 6. DETAILED DESCRIPTION

### 6.1 Definitions

As used herein, the following terms are intended to have the following meanings:

The term "antibody" refers to an immunoglobulin molecule that specifically binds to, or is immunologically reactive with, a particular antigen, and includes polyclonal, monoclonal, genetically engineered (e.g., rIgG) and otherwise modified forms of antibodies, including but not limited to chimeric antibodies, humanized antibodies, heteroconjugate antibodies (including, *e.g*., bispecific antibodies), antigen binding fragments of antibodies, including *e.g*., Fab', F(ab')₂, Fab, Fv, and scFv fragments and multimerici forms of antigen binding fragments, including e.g., diabodies, triabodies and tetrabodies. Moreover, unless otherwise indicated, the term "monoclonal antibody" (mAb) is meant to include both intact molecules, as well as, antibody fragments (such as, for example, Fab and F(ab')₂ fragments) which are capable of specifically binding to a protein. Fab and F(ab')₂ fragments lack the Fc fragment of intact antibody, clear more rapidly from the circulation of the animal or plant, and may have less non-specific tissue binding than an intact antibody (Wahl et al., 1983, J. Nucl. Med. 24:316).

The term "scFv" refers to a single chain Fv antibody in which the variable domains of the heavy chain and the light chain from a traditional antibody have been joined to form one chain.

References to "VH" refer to the variable region of an immunoglobulin heavy chain of an antibody, including the heavy chain of an Fv, scFv, or Fab. References to "VL" refer to the variable region of an immunoglobulin light chain, including the light chain of an Fv, scFv, dsFv or Fab. Antibodies (Abs) and immunoglobulins (Igs) are glycoproteins having the same structural characteristics. While antibodies exhibit binding specificity to a specific target, immunoglobulins include both antibodies and other antibody-like molecules which lack target specificity. Native antibodies and immunoglobulins are usually heterotetrameric glycoproteins of about 150,000 daltons, composed of two identical light (L) chains and two identical heavy (H) chains. Each heavy chain has at one end a variable domain (VH) followed by a number of constant domains. Each light chain has a variable domain at one end (VL) and a constant domain at its other end.

Complementarity determining regions (CDRs) are also known as hypervariable regions both in the light chain and the heavy chain variable domains. The more highly conserved portions of variable domains are called the framework (FR). As is known in the art, the amino acid position/boundary delineating a hypervariable region of an antibody can vary, depending on the context and the various definitions known in the art. Some positions within a variable domain may be viewed as hybrid hypervariable positions in that these positions can be deemed to be within a hypervariable region under one set of criteria while being deemed to be outside a hypervariable region under a different set of criteria. One or more of these positions can also be found in extended hypervariable regions. The disclosure provides antibodies comprising modifications in these hybrid hypervariable positions. The variable domains of native heavy and light chains each comprise four FR regions, largely by adopting a β-sheet configuration, connected by three CDRs, which form loops connecting, and in some cases forming part of, the β-sheet structure. The CDRs in each chain are held together in close proximity by the FR regions and, with the CDRs from the other chain, contribute to the formation of the target binding site of antibodies (See Kabat et al., Sequences of Proteins of Immunological Interest (National Institute of Health, Bethesda, Md. 1987). As used herein, numbering of immunoglobulin amino acid residues is done according to the immunoglobulin amino acid residue numbering system of Kabat *et al.,* unless otherwise indicated.

The term "antibody fragment" refers to a portion of a full-length antibody, generally the target binding or variable region. Examples of antibody fragments include Fab, Fab', F(ab')2 and Fv fragments. An "Fv" fragment is the minimum antibody fragment which contains a complete target recognition and binding site. This region consists of a dimer of one heavy and one light chain variable domain in a tight, noncovalent association (VH -VL dimer). It is in this configuration that the three CDRs of each variable domain interact to define a target binding site on the surface of the VH -VL dimer. Collectively, the six CDRs confer target binding specificity to the antibody. However, even a single variable domain (or half of an Fv comprising only three CDRs specific for a target) has the ability to recognize and bind target, although at a lower affinity than the entire binding site. "Single-chain Fv" or "scFv" antibody fragments comprise the VH and VL domains of an antibody, wherein these domains are present in a single polypeptide chain. Generally, the Fv polypeptide further comprises a polypeptide linker between the VH and VL domains which enables the scFv to form the desired structure for target binding.

The Fab fragment contains the constant domain of the light chain and the first constant domain (CH1) of the heavy chain. Fab' fragments differ from Fab fragments by the addition of a few residues at the carboxyl terminus of the heavy chain CH1 domain including one or more cysteines from the antibody hinge region. Fab' fragments are produced by cleavage of the disulfide bond at the hinge cysteines of the F(ab')₂ pepsin digestion product. Additional chemical couplings of antibody fragments are known to those of ordinary skill in the art.

"Epitope" or "antigenic determinant" refers to a site on an antigen to which an antibody binds. Epitopes can be formed both from contiguous amino acids or noncontiguous amino acids juxtaposed by tertiary folding of a protein. Epitopes formed from contiguous amino acids are typically retained on exposure to denaturing solvents whereas epitopes formed by tertiary folding are typically lost on treatment with denaturing solvents. An epitope typically includes at least 3, and more usually, at least 5 or 8-10 amino acids in a unique spatial conformation. Methods of determining spatial conformation of epitopes include, for example, x-ray crystallography and 2-dimensional nuclear magnetic resonance. See, e.g., Epitope Mapping Protocols in Methods in Molecular Biology, Vol. 66, Glenn E. Morris, Ed (1996).

The determination of whether two antibodies bind substantially to the same epitope is accomplished using methods known in the art, such as a competition assay. In conducting an antibody competition study between a control antibody (for example, daclizumab) and any test antibody, one may first label the control antibody with a detectable label, such as, biotin, an enzyme, radioactive label, or fluorescent label to enable the subsequent identification. In such an assay, the intensity of bound label is measured in a sample containing the labeled control antibody and the intensity of bound label sample containing the labeled control antibody and the unlabeled test antibody is measured. If the unlabeled test antibody competes with the labeled antibody by binding to an overlapping epitope, the detected label intensity will be decreased relative to the binding in the sample containing only the labeled control antibody. Other methods of determining binding are known in the art.

The term "monoclonal antibody" refers to an antibody that is derived from a single clone, including any eukaryotic, prokaryotic, or phage clone, and not the method by which it is produced. Antibodies immunologically reactive with a particular antigen can be prepared using a wide variety of techniques known in the art including the use of hybridoma, recombinant, and phage display technologies, or a combination thereof. For example, monoclonal antibodies can be produced using hybridoma techniques including those known in the art and taught, for example, in Harlow and Lane, "Antibodies: A Laboratory Manual," Cold Spring Harbor Laboratory Press, New York (1988); Hammerling et al., in: "Monoclonal Antibodies and T-Cell Hybridomas," Elsevier, N.Y. (1981), pp. 563 681 (both of which are incorporated herein by reference in their entireties).

A "chimeric antibody" is an immunoglobulin molecule in which (a) the constant region, or a portion thereof, is altered, replaced or exchanged so that the antigen binding site (variable region) is linked to a constant region of a different or altered class, effector function and/or species, or an entirely different molecule which confers new properties to the chimeric antibody, e.g., an enzyme, toxin, hormone, growth factor, drug, etc.; or (b) the variable region, or a portion thereof, is altered, replaced or exchanged with a variable region having a different or altered antigen specificity. Any of the anti-IL-2R antibodies described herein can be chimeric.

The term "humanized antibody" or "humanized immunoglobulin" refers to an immunoglobulin comprising a human framework, at least one and preferably all complementarity determining regions (CDRs) from a non-human antibody, and in which any constant region present is substantially identical to a human immunoglobulin constant region, i.e., at least about 85%, at least 90%, and at least 95% identical. Hence, all parts of a humanized immunoglobulin, except possibly the CDRs, are substantially identical to corresponding parts of one or more native human immunoglobulin sequences. Often, framework residues in the human framework regions will be substituted with the corresponding residue from the CDR donor antibody to alter, preferably improve, antigen binding. These framework substitutions are identified by methods well known in the art, e.g., by modeling of the interactions of the CDR and framework residues to identify framework residues important for antigen binding and sequence comparison to identify unusual framework residues at particular positions. See, e.g., Queen et al., U.S. Pat. Nos: 5,530,101; 5,585,089; 5,693,761; 5,693,762; 6,180,370 (each of which is incorporated by reference in its entirety). Antibodies can be humanized using a variety of techniques known in the art including, for example, CDR-grafting (EP 239,400; PCT publication WO 91/09967; U.S. Pat. Nos. 5,225,539; 5,530,101 and 5,585,089), veneering or resurfacing (EP 592,106; EP 519,596; Padlan, Mol. Immunol., 28:489 498 (1991); Studnicka et al., Prot. Eng. 7:805 814 (1994); Roguska et al., Proc. Natl. Acad. Sci. USA, 91:969 973 (1994), and chain shuffling (U.S. Pat. No. 5,565,332), all of which are hereby incorporated by reference in their entireties. The anti-IL-2R antibodies described herein include humanized antibodies, such as mouse humanized antibodies, fully human antibodies, and mouse antibodies.

An "anti-IL-2R antibody" is an antibody that specifically binds an IL-2 receptor. For example, in some embodiments, an anti-IL-2R antibody binds the high affinity IL-2 receptor (K_{d} ~ 10 pM). This receptor is a membrane receptor complex consisting of the two subunits: IL-2R-alpha (also known as T cell activation (Tac) antigen, CD25, or p55) and IL-2R-beta (also known as p75 or CD122). In other embodiments, an anti-IL-2R antibody binds the intermediate affinity IL-2 receptor (K_{d} = 100 pM), which consists of the p75 subunit and a gamma chain. In other embodiments, an anti-IL-2R antibody binds the low affinity receptor (K _{d} = 10 nM), which is formed by p55 alone.

Anti-IL-2R antibodies suitable for use in the methods described herein include monoclonal antibodies, chimeric antibodies, humanized antibodies, or fully human antibodies. Examples of anti-IL-2R antibodies capable of binding Tac (p55) include, but are not limited to, Zenapax®, the chimeric antibody basiliximab (Simulect®), BT563 (see Baan et al., Transplant. Proc. 33:224-2246, 2001), and 7G8, and HuMax-TAC being developed by Genmab. The mik-beta1 antibody specifically binds the beta chain of human IL-2R. Additional antibodies that specifically bind the IL-2 receptor are known in the art. For example, see U.S. Pat. No. 5,011,684; U.S. Pat. No. 5,152,980; U.S. Pat. No. 5,336,489; U.S. Pat. No. 5,510,105; U.S. Pat. No. 5,571,507; U.S. Pat. No. 5,587,162; U.S. Pat. No. 5,607,675; U.S. Pat. No. 5,674,494; U.S. Pat. No. 5,916,559.

### 6.2 Detailed Description

MS is an inflammatory/demyelinating disease of the CNS that is one of the leading causes of neurological disability in young adults (Bielekova, B. and Martin, R., 1999, Curr Treat Options Neurol. 1:201-219). The pathogenesis observed in MS patients is, at least in part, attributable to aberrant T-cell activation. Daclizumab is a humanized antibody that binds the IL-2R alpha chain (also known as T cell activation (TAC) antigen, CD25 or p55). CD25 is present at low levels in resting human T cells, but is significantly up-regulated on activated T cells, enabling them to receive a high-affinity IL-2 signal (Waldmann, et al., 1998, Int Rev Immunol, 16:205-226). It has been proposed that selective binding of CD25 by daclizumab inhibits IL-2R signal transduction events (Goebel, J., et al., 2000, Transplant Immunol, 8:153-159, Tkaczuk, J., 2001, Transplant Proc, 33:212-213) and blocks T cell activation (Queen, C., et al., 1989, Proc Natal Acad Sci USA, 86:10029-10033).

The methods described herein are based upon the discovery that rapid reductions in HLA-DR⁺CD4⁺ activated T cells are observed in MS patients treated with daclizumab (see FIG. 2). Rapid reductions in activated T cell numbers is consistent with reductions in Gd-CEL observed in daclizumab treated MS patients (Montalban, X. et al., Multiple Sclerosis, 13: S18-S18 Suppl. 2 OCT 2007; and, Kaufman, M.D., et al., Neurology, 70 (11): A220-A220 Suppl. 1 MAR 11 2008). These observations suggest that changes in the level of HLA-DR⁺CD4⁺ activated T cells would be a useful tool for monitoring the clinical response in MS patients to daclizumab or an anti-IL-2R antibody capable of inhibiting IL-2R signal transduction events.

A number of studies have investigated HLA-DR⁺ expression in blood samples obtained from patients with autoimmune diseases, including MS, to determine if HLA-DR⁺ expression could be used to monitor disease progression during drug therapy. None of these studies have demonstrated that HLA-DK⁺ expression could be used to monitor disease progression in response to drug therapy. For example, Ferrarini et al., monitored the expression of HLA-DR and CD25 on T cells, as well as circulating CD3⁺HLA-DR⁺ and CD4⁺CD25⁺ cells in relapsing-remitting multiple sclerosis patients during interferon beta 1b therapy and determined that the expression of HLA-DR could not be used to monitor MS activity during IFN beta 1b therapy (Ferrarini et al., 1998, Multiple Sclerosis, 4:174-177). Other studies have also tried to establish a link between the expression of T-cell activation markers including HLA-DR and disease activity and/or drug therapy in MS (see, for example, Tang, M. T., et al., 2008, Multiple Sclerosis, 14:S181-S181 Suppl. 1; Aristimuno, C., et al., 2008, Journal Neuroimmunology, 204:131-135; Heinrich, A., et al., 2006, Acta Neurol Scand, 113:248-255; Sanchez-Ramon, S., et al., 2005, Immunology Letters, 96:195-201; Gelati, M., et al., 1999, J Neurol, 246:569-573; Genc, K., et al., 1997, J Clin Invest, 1:2664-2671; and Bever, C., et al., 1991, Int J Immunopharmac, 13:613-618).

Bielekova et al., reported that in MS patients treated with daclizumab there was a gradual decline in circulating CD4⁺ and CD8⁺ T cells and significant expansion of CD56^{bright} natural killer (NK) cells in vivo and this effect correlated highly with the response to daclizumab (Bielekova, B., et al., 2006, Proc Natl Acad Sci USA, 103:5941-5946). The positive correlations between expansion of CD56^{bright} NK cells and contraction of CD4⁺ and CD8⁺ T cell numbers was proposed to support the existence of an immunoregulatory pathway wherein activated CD56^{bright} NK cells inhibit T cell survival (Bielekova, B., et al., 2006, Proc Natl Acad Sci USA, 103:5941-5946).

Although Bielekova et. al. (Bielekova, B., et al., 2006, Proc Natl Acad Sci USA, 103:5941-5946) reported statistically significant reductions from baseline in total CD4+ T cell counts (mean = -11.2%, p = 0.009) when MS patients (n = 22) were treated with daclizumab in combination with IFN beta therapy, the observation of reduced levels of CD4⁺ T cell counts associated with daclizumab treatment were not reproduced during the placebo-controlled, blinded, CHOICE study. During the CHOICE study CD4⁺ T cell counts were obtained as often as 10 time points for each subject over the course of the 44 week study, from approximately 65 subjects, and statistically significant percent changes from baseline (parametric analysis within treatment groups) (p<0.05) were limited to a reduction (mean = 18.2%) in the placebo treatment group at Week 2 (n = 16), and a reduction (mean = 19.1 %) in the high dose daclizumab treatment group at Week 12 (n = 19); there was no significant change for the low dose daclizumab treatment group.

As shown in Table 1, the observed reductions in CD4⁺ T cell counts were incidental and showed no treatment related trend. The P values shown in Table 1 were obtained from an ANOVA comparison to IFN-beta + Placebo group absolute cell counts at Week 22 of the treatment phase, and thus changes from baseline levels in CD4⁺ T cell counts observed in the two daclizumab treatment groups of the CHOICE study were not statistically significant in comparison to placebo group and were deemed to be incidental. Shown in parenthesis (n) is the number of patients sampled within each treatment group.

**Table 1. Daclizumab associated changes in CD4⁺ T cell counts**

| **Immune Subset** | **Treatment** | **Baseline cells/mm³ median** | **Week 22 cells/mm³ median** | **Week 22 % change median** | **Week 22 P value (n)** |
|---|---|---|---|---|---|
| CD4⁺ T cells | IFN-beta + Placebo | 735 | 606 | -5.2 | (13) |
| | IFN -beta + DAC 1 mg/kg | 789 | 697 | -16.8 | 0.74 (18) |
| | IFN -beta + DAC 2 mg/kg | 652 | 546 | -20.9 | 0.82 (19) |
| CD4⁺HLA-DR⁺ | IFN -beta + Placebo | 31.2 | 32.2 | -7.3 | (13) |
| | IFN -beta + DAC 1 mg/kg | 31.1 | 20.2 | -45.7 | 0.006(18) |
| | IFN -beta + DAC 2 mg/kg | 27.6 | 20.3 | -32.7 | 0.009(17) |

Moreover, the HLA-DR⁺CD4⁺ T cell subset typically represents a small percentage of all CD4⁺ T cells present in both healthy and diseased human subjects. The range of median percentage HLA-DR⁺CD4⁺ T cells for placebo subjects at all time points monitored during the CHOICE study was 5.5% to 6.3% of all CD4+ T cells. The maximum mean reductions from baseline in HLA-DR⁺CD4⁺ T counts as determined during the CHOICE study, mean max reduction daclizumab low dose group = -38.9%, mean max reduction daclizumab high dose group = -48.4%, were far greater than the -11.2% change in total CD4⁺ T cells reported by Bielekova et. al. (Bielekova, B., et al., 2006, Proc Natl Acad Sci USA, 103:5941-5946). The magnitude of change observed in HLA-DR⁺CD4⁺ T cell counts in response to treatment with daclizumab in the CHOICE study, supports the use of HLA-DR⁺CD4⁺ T cell counts as a sensitive marker for monitoring the efficacy of anti-IL-2R antibody therapy in MS patients.

Described herein are methods for the use of HLA-DR⁺CD4⁺ T cells as a marker for anti-IL2R antibody activity in MS patients. Typically, blood samples from MS patients are analyzed for cell surface markers using flow cytometry (see, e.g., Bielekova, B., et al., 2006, Proc Natl Acad Sci USA, 103:5941-5946). For example, the number of HLA-DR⁺CD4⁺ T cells can be analyzed using commercially available antibodies that bind preferentially to the HLA-DR⁺ protein, and in combination with fluorescent activated cell sorting (FACS), the levels of HLA-DR+ expressing cells determined. The percent change in the number of HLA-DR⁺CD4⁺ T cells in response to treatment can be used to monitor the efficacy of an anti-IL-2R antibody.

In some embodiments, a reduction in the number of HLA-DR⁺CD4⁺ T cells is observed in response to treatment with an IL-2R antibody. The number of HLA-DR⁺CD4⁺ T cells can be determined by absolute cell count, i.e., the number of cells per mm³ or mm², or as a percent of total CD4+ T cells. Depending on the individual patient and the relapsing form of MS, the overall reduction in the number of HLA-DR⁺CD4⁺ T cells can vary from 25% to 100%. For example in some embodiments, the number of HLA-DR⁺CD4⁺ T cells can be reduced by at least 25%, by at least 30%, by at least 35%, by at least 40%, by at least 45%, by at least 50%, by at least 55%, by at least 60%, by at least 65%, by at least 70%, by at least 75%, by at least 80%, by at least 85%, by at least 90%, by at least 95%, or by at least 100%.

In some embodiments, additional cell surface markers can be monitored to provide additional information regarding the clinical response in MS patients treated with an anti-IL-2R antibody. Additional cell surface markers include, but are not limited to, CD3, CD56^{bright} NK cells, CD4, CD25, CD16, CD122, and CD8. Assays for the determination of these markers have been described, see, e.g., Bielekova, B., et al., 2006, Proc Natl Acad Sci USA, 103:5941-5946. For example, in some embodiments, the number of HLA-DR⁺CD4⁺ T cells and the number of CD56^{bright} NK cells can be analyzed and used to monitor the efficacy of an anti-IL-2R antibody.

The determination of the number of HLA-DR⁺CD4⁺ T cells generally requires that more than one sample be taken from a patient at selected times. The determination of sampling time is not critical to the methods described herein and can be selected by a medical practitioner based, in part on the length of time a patient has been treated with an anti-IL-2R antibody. Other factors that can affect sampling time include, but are not limited to, the length of time the patient has been treated for MS, which therapy(s) the patient has received prior to treatment with an anti-Il-2R antibody, and whether the patient is showing one or more of the following symptoms: increased relapse rate, an increase in the Expanded Disability Status Scale (EDSS) score, an increased number of new or enlarged Gd-CEL, and an increase in new or enlarged T2 MRI lesions. See, e.g., Perini et al., 2004, J Neurology, 251:305-309; Sorensen, et al., 2003, Lancet, 362:184-191; Pachner, 2003, Neurology, 61 (Suppl 5):S2-S5; Perini et al., 2004, J Neurology, 251:305-309; and Farrell, et al., 2008, Multiple Sclerosis, 14:212-218.

For example, in some embodiments, changes in the number of HLA-DR⁺CD4⁺ T cells can be monitored before, during and after initiation of treatment with an anti-IL-2R antibody.

By way of another example, changes in the number of HLA-DR⁺CD4⁺ T cells can be monitored prior to the initiation of treatment with an anti-IL-2R antibody and at selected intervals thereafter (e.g., weekly, monthly, once every two months, once every three months, once every six months).

By way of another example, changes in the number of HLA-DR⁺CD4⁺ T cells can be monitored following initiation of treatment (e.g., within a week, within a month, within two months, within three months, within six months) with an anti-IL-2R antibody and at selected intervals thereafter (e.g., weekly, monthly, once every two months, once every three months, once every six months).

Typically, a 25 % or greater reduction in the number of HLA-DR⁺CD4⁺ T cells is consistent with the administration of a therapeutically effective dose of an anti-IL-2R antibody. As used herein, a "therapeutically effective dose" is a dose sufficient to prevent advancement, decrease the relapse rate, or reduce one or more of the symptoms associated with disease progression in multiple sclerosis.

For example, in some embodiments administration of a therapeutically effective dose of an anti-IL-2R antibody to a MS patient decreases the number of relapses by at least one that occur in a given time period, such as 1 year, in the treated patient. Relapses are typically assessed by history and physical examination defined as the appearance of a new symptom or worsening of an old symptom attributable to multiple sclerosis, accompanied by an appropriate new neurological abnormality or focal neurological dysfunction lasting at least 24 hours in the absence of fever, and preceded by stability or improvement for at least 30 days (see, e.g., Sorensen, et al., 2003, Lancet, 362: 1184-1191.

In other embodiments, administration of a therapeutically effective dose of an anti-IL-2R antibody to a MS patient decreases the number of lesions detected in the patient's brain. Magnetic Resonance Imaging (MRI) of the brain is an important tool for understanding the dynamic pathology of multiple sclerosis. T₂-weighted brain MRI defines lesions with high sensitivity in multiple sclerosis and is used as a measure of disease burden. However, such high sensitivity occurs at the expense of specificity, as T₂ signal changes can reflect areas of edema, demyelination, gliosis and axonal loss. Areas of Gd-CEL demonstrated on T₁-weighted brain MRI are believed to reflect underlying blood-brain barrier disruption from active perivascular inflammation. Such areas of enhancement are transient, typically lasting <1 month. Gd-CEL brain MRI is therefore used to assess disease activity. Most T₂-weighted (T2) lesions in the central white matter of subjects with multiple sclerosis begin with a variable period of Gd-CEL. Gd-CEL and T2 lesions represent stages of a single pathological process. Brain MRI is a standard technique for assessing Gd-CEL and T2 lesions and is routinely used to assess disease progression in MS (e.g., see Lee et al., Brain 122 (Pt 7):1211-2, 1999).

As shown in FIG 1, treatment of MS patients with daclizumab reduced the mean number of new and enlarged Gd-CEL by 25% or more. Accordingly, in some embodiments a therapeutically effective dose of an anti-IL-2R antibody to a MS patient decreases the number of Gd-CEL detected in the patient's brain by approximately 25% to 80%. In some embodiments, the number of Gd-CEL detected in the patient's brain is decreased by at least 25%, by at least 30%, by at least 35%, by at least 40%, by at least 45%, by at least 50%, by at least 55%, by at least 60%, by at least 65%, by at least 70%, by at least 75%, by at least 80%, by at least 85%, by at least 90%, by at least 95%, and by at least 100%.

In other embodiments, administration of a therapeutically effective dose of an anti-IL-2R antibody to a MS patient decreases the number of T2 MRI lesions detected in the patient's brain.

In other embodiments, administration of a therapeutically effective dose of an anti-IL-2R antibody to a MS patient stabilizes a patient's disability progression as determined by the "Expanded Disability Status Scale (EDSS)" which can be used to rate neurological impairment in MS patients (Kurtzke, 1983, Neurology, 33-1444-52). The EDSS comprises 20 grades from 0 (normal) to 10 (death due to MS), progressing in a single-point step from 0 to1 and in 0.5 point steps upward. The scores are based on a combination of functional-system scores, the patient's degree of mobility, need for walking assistance, or help in the activities of daily living. The functional-system scores measure function within individual neurological systems including visual, pyramidal, cerebellar, brainstem, sensory, bowel and bladder, cerebral and other functions.

In other embodiments, administration of a therapeutically effective dose of an anti-IL-2R antibody to an IFN-beta NAb positive MS patient reduces a patient's disability score by 10% to 75%. For example in some embodiments, a patient's disability score can be reduced by at least 10%, by at least 15%, by at least 20%, by at least 25%, by at least 30%, by at least 35%, by at least 40%, by at least 45%, by at least 50%, by at least 55%, by at least 60%, by at least 65%, by at least 70%, or by at least 75%.

In some embodiments, no change or an increase in the number of HLA-DR⁺CD4⁺ T cells following treatment of a MS patient with an anti-IL-2R antibody will be observed. In these embodiments, the patients can be assessed to determine if they are responding poorly or failing to respond to treatment with an anti-IL-2R antibody. MS patients that are responding poorly to therapy generally have a higher mean relapse rate, a higher risk of experiencing a second relapse, a higher risk of having a sustained progression of ≥ 1 on EDSS, and a lower probability of being relapse free (Malucchi, et al., 2004, Neurology, 62: 2031-2037). Accordingly, a number of clinical endpoints can be used to determine whether a patient is responding to treatment with an anti-IL-2R antibody including the frequency and rate of relapse, a 1 point or greater increase in the Expanded Disability Status Scale (EDSS) score, an increase in the number of Gd-CEL, and/or an increase in the number of T2 MRI lesions.

The data required to determine clinical endpoints can be collected at the start of the anti-IL-2R treatment and/or during follow-up visits. In some embodiments, MS patients that are responding poorly to an anti-IL-2R antibody can be treated with additional agents. For example, in some embodiments, one or more anti-IL-2R antibodies can be administered to a MS patient. In other embodiments, an anti-IL-2R antibody can be administered in combination with another MS therapy, such as an IFN-beta product. Examples of suitable IFN-beta products include, but are not limited to, one of the three IFN-beta products that have been approved: IFN-beta-1b (Betaferon®, Schering AG, Berlin, Germany), IFN-beta-1a (Avonex®, Biogen Idec, Cambridge MA), and IFN-beta-1a (Rebif®, Ares-Serono, Geneva, Switzerland). Non-limiting examples of other marketed drugs that may be used in combination with an anti-IL-2R treatment include glatiramer acetate (e.g., Copaxone®, Teva Pharmaceutical Industries, Ltd., Israel), corticosteroids, riluzole, azathioprine, cyclophosphamide, methotrexate, and mitoxantrone.

MS patients suitable for treatment with an anti-IL-2R antibody typically have been diagnosed with a relapsing form of multiple sclerosis including relapsing-remitting multiple sclerosis, secondary progressive multiple sclerosis, progressive relapsing multiple sclerosis and worsening relapsing multiple sclerosis. By "relapsing-remitting multiple sclerosis" herein is meant a clinical course of MS that is characterized by clearly defined, acute attacks with full or partial recovery and no disease progression between attacks. By "secondary-progressive multiple sclerosis" herein is meant a clinical course of MS that initially is relapsing-remitting, and then becomes progressive at a variable rate, possibly with an occasional relapse and minor remission. By "progressive relapsing multiple sclerosis" herein is meant a clinical course of MS that is progressive from the onset, punctuated by relapses. There is significant recovery immediately following a relapse, but between relapses there is a gradual worsening of disease progression. By "worsening relapsing multiple sclerosis" herein is meant a clinical course of MS with unpredictable relapses of symptoms, from which people do not return to normal and do not recover fully.

In some embodiments, the anti-IL-2 receptor antibody is daclizumab. The recombinant genes encoding daclizumab are a composite of human (about 90%) and murine (about 10%) antibody sequences. The donor murine anti-Tac antibody is an IgG2a monoclonal antibody that specifically binds the IL-2R Tac protein and inhibits IL-2-mediated biologic responses of lymphoid cells. The murine anti-Tac antibody was "humanized" by combining the complementarity-determining regions and other-selected residues of the murine anti-TAC antibody with the framework and constant regions of the human IgG1 antibody. The humanized anti-Tac antibody daclizumab is described and its sequence is set forth in U.S. Pat. No. 5,530,101, see SEQ ID NO: 5 and SEQ ID NO: 7 for the heavy and light chain variable regions respectively. SEQ ID NOS: 5 and 7 of US Pat. No. 5,530,101 are disclosed as SEQ ID NOS: 1 and 2 respectively in the sequence listing filed herewith. U.S. Pat. No. 5,530,101 and Queen et al., Proc. Natl. Acad. Sci. 86:1029-1033, 1989 are both incorporated by reference herein in their entirety.

Daclizumab has been approved by the U.S. Food and Drug Administration (FDA) for the prophylaxis of acute organ rejection in subjects receiving renal transplants, as part of an immunosuppressive regimen that includes cyclosporine and corticosteroids and is marketed by Roche as ZENAPAX®. Daclizumab also has been shown to be active in the treatment of human T cell lymphotrophic virus type 1 associated myelopathy/topical spastic paraparesis (HAM/TSP, see Lehky et al., Ann. Neuro., 44:942-947, 1998). The use of daclizumab to treat posterior uveitis has also been described (see Nussenblatt et al., Proc. Natl. Acad. Sci., 96:7462-7466, 1999).

Antibodies that bind the same (or overlapping) epitope as daclizumab can be used in the methods disclosed herein. In some embodiments, the antibody will have at least 90%, at least 95%, at least 98%, or at least 99% sequence identity with daclizumab. The antibody can be of any isotype, including but not limited to, IgG1, IgG2, IgG3 and IgG4.

In some embodiments, the antibody is basiliximab, marketed as Simulect® by Novartis Pharma AG. Basiliximab is a chimeric (murine/human) antibody, produced by recombinant DNA technology that functions as an immunosuppressive agent, specifically binding to and blocking the alpha chain of the IL-2R on the surface of activated T-lymphocytes.

Anti-IL-2R antibodies can be administered parenterally, i.e., subcutaneously, intramuscularly, intravenously, intranasally, transdermally, or by means of a needle-free injection device. The compositions for parenteral administration will commonly include a solution of an anti-IL-2R antibody in a pharmaceutically acceptable carrier. Pharmaceutically-acceptable, nontoxic carriers or diluents are defined as vehicles commonly used to formulate pharmaceutical compositions for animal or human administration. See, for example, Remington: The Science and Practice of Pharmacy, A.R. Gennaro, 20th Edition, 2001, Lippincott Williams & Wilkins, Baltimore, MD, for a description of compositions and formulations suitable for pharmaceutical delivery of the anti-IL-2R antibodies disclosed herein. See US Pat. Appl. Pub. Nos. 2003/0138417 and 2006/0029599 for a description of liquid and lyophilized formulations suitable for the pharmaceutical delivery of daclizumab.

Methods for preparing pharmaceutical compositions are known to those skilled in the art (see Remington: The Science and Practice of Pharmacy, *supra*). In addition, the pharmaceutical composition or formulation can include other carriers, adjuvants, or nontoxic, non-therapeutic, nonimmunogenic stabilizers and the like. Effective amounts of such diluent or carrier will be those amounts that are effective to obtain a pharmaceutically acceptable formulation in terms of solubility of components, or biological activity.

The concentration of antibody in the formulations can vary widely, i.e., from less than about 0.5%, usually at or at least about 1% to as much as 15 or 20% by weight or from 1 mg/mL to 100 mg/mL. The concentration is selected primarily based on fluid volumes, viscosities, etc., in accordance with the particular mode of administration selected.

Generally a suitable therapeutic dose of daclizumab is about 0.5 milligram per kilogram (mg/kg) to about 5 mg/kg, such as a dose of about 0.5 mg/kg, of about 1 mg/kg, about 1.5 mg/kg, about 2 mg/kg, about 2.5 mg/kg, about 3.0 mg/kg, about 3.5 mg/kg, about 4.0 mg/kg, about 4.5 mg/kg, or about 5.0 mg/kg administered intraveneously or subcutaneously. Unit dosage forms are also possible, for example 50 mg, 100 mg, 150 mg, 200 mg, 300 mg, 400 mg, or up to 500 mg per dose.

Other dosages can be used to obtain serum levels of 2 to 5 µg/mL which are necessary for saturation of the Tac subunit of the IL-2 receptor to block the responses of activated T lymphocytes. Higher levels such as approximately 5 to 40 µg/mL, may be necessary for clinical efficacy. One of skill in the art will be able to construct an administration regimen to keep serum levels within the 2 to 40 µg/mL range.

Doses of basiliximab are likely to be lower, for example 0.25 mg/kg to 1 mg/kg, e.g., 0.5 mg/kg, or unit doses of 10, 20, 40, 50 or 100 mg, due to the higher affinity of basiliximab for the IL-2R target. The general principle of keeping the IL-2 receptor saturated can be used to guide the choice of dose levels of other IL-2R antibodies.

Single or multiple administrations of anti-IL-2R antibodies can be carried out with dosages and frequency of administration selected by the treating physician. Generally, multiple doses are administered. For example, multiple administration of daclizumab or other anti-IL-2R antibodies can be utilized, such as administration monthly, bimonthly, every 6 weeks, every other week, weekly or twice per week.

### 7. EXAMPLES

### Example 1: CHOICE Study

The CHOICE study was a Phase 2, randomized, double-blinded, placebo-controlled, multi-center study of subcutaneous (SC) daclizumab added to interferon (IFN)-beta in the treatment of active, relapsing forms of MS. Results from the CHOICE study confirmed that daclizumab at 2 mg/kg every two weeks significantly decreased the number of new Gd-CEL in patients who have active, relapsing forms of MS on concurrent IFN-beta therapy (Montalban, X. et al., Multiple Sclerosis, 13: S18-S18 Suppl. 2 OCT 2007; and, Kaufman, M.D., et.al., Neurology, 70 (11): A220-A220 Suppl. 1 MAR 11 2008). A smaller decrease in new or enlarging Gd-CEL was observed for those study subjects receiving 1 mg/kg daclizumab every four weeks.

A patient was enrolled in the study once he or she was randomized. Enrolled patients remained on their baseline IFN-beta regimen and were randomized in a 1:1:1 ratio to one of the following 3 treatment arms (see Table 2).

**Table 2**

| **Treatment Arm¹** | **Dose Level and Frequency** | **No. Total Dosing Visits** | **No. Patient s** |
|---|---|---|---|
| A (High Dose)² | Daclizumab SC: 2 mg/kg q2weeks x 11 doses | 11 | 55 |
| B (Low Dose)³ | Daclizumab SC: 1 mg/kg q4 weeks x 6 doses | 11 | 55 |
| C (Placebo)⁴ | Placebo SC: q2 weeks x 11 doses | 11 | 55 |

| | | | |
|---|---|---|---|
| 1 All patients continue on prior regimen of IFN-beta SC/IM for the duration of the study. 2 Patients in Arm A (high dose) receive 2 SC injections (2 daclizumab 1 mg/kg) for 11 dosing visits. Maximum dose daclizumab per dosing visit = 200 mg. 3 Patients in Arm B (low dose) receive 2 SC injections (1 daclizumab 1 mg/kg, 1 placebo) for 6 dosing visits, alternating with 2 SC injections (2 placebo) for 5 dosing visits. Maximum dose daclizumab per daclizumab dosing visit = 100 mg. 4 Patients in Arm C (placebo) receive 2 SC injections (2 placebo) for 11 dosing visits. | | | |

The screening period was up to 3 weeks. The treatment period was designated as 24 weeks (6 months, through Day 168) in order to include 4 weeks subsequent to the last dose of blinded study drug (Dose No. 11, which occurs at Visit No. 14, Day 140). After the treatment period, patients were followed for a total of 48 weeks (12 months) and continued IFN beta therapy for at least 5 months of this period. Total maximum time on study for each patient was approximately 18 months.

Evaluations of a given patient by EDSS and Multiple Sclerosis Functional Composite, version 3 (MSFC-3) were performed by a clinician who was not involved in the patient's treatment and was designated an "evaluating clinician." All other assessments of the patient were under the purview of the clinician in charge of the patient's treatment (treating clinician). The MSFC-3 includes quantitative tests of: (1) Leg function/ambulation-Timed 25-foot walk (T25FW); (2) Arm function-9-Hole Peg Test (9HPT), and (3) Cognition-Paced Auditory Serial Addition Test with 3-second interstimulus intervals (PASAT3) (Cutter et al., 1999, Brain, 122(Pt 5):871-882).

Preliminary eligibility for the CHOICE study was established by history, chart inspection, and routine evaluations. During the treatment and follow up period, a number of procedures and evaluations were performed on the subjects at specified days including, but not limited to, MRI, EDSS, MSFC-3, physical exams, symptom directed physical exams, hematology/serum chemistry (e.g., for determination of pharmacokinetic assessment and anti-DAC antibodies), and blood draws for pharmacodynamic assessments and IFN-beta NAbs.

Daclizumab drug substance manufactured by PDL BioPharma, Inc. (Redwood City, CA) for subcutaneous delivery, was supplied in single-use vials containing 100 mg of daclizumab in 1.0 mL of 40 mM sodium succinate, 100 mM sodium chloride, 0.03% polysorbate 80, pH 6.0. Placebo was supplied in single-use vials as an isotonic solution in matching vials containing 40 mM sodium succinate, 6% sucrose, 0.03% polysorbate 80, pH 6.0.

### Example 2: Reductions in HLA-DR⁺CD4⁺ T cells in Response to Treatment with Daclizumab

Changes in the number HLA-DR⁺CD4⁺ T cells were determined in patients that participated in the CHOICE study (see Example 1). HLA-DR⁺CD4⁺ T cell levels were analyzed in patients that received daclizumab 2 mg/kg every 2 weeks (high dose), daclizumab 1 mg/kg every 4 weeks (low dose), or placebo SC added to interferon beta therapy for 24 weeks. Patient's whole blood specimens were collected into vacutainer tubes containing anti-coagulant using standard venipuncture techniques and shipped via overnight courier for immediate, treatment-blinded, analysis using fluorescent activated cell sorting (FACS). Commercially available antibodies were obtained from BD Biosciences (USA) that bind preferentially to the HLA-DR protein (e.g., antibody clone L243(G45-6)) and that bind preferentially to specific immune subsets (e.g. antibodies that bind selectively to CD3 (e.g., clone SK7) and CD4 (e.g., clone SK3) antigens were used in combination with FACS to determine levels of HLA-DR+ expressing cells when patients were treated with DAC antibody. Specimen preparation and FACS assay procedures were performed in accordance with the practices recommended in the "Guideline for Flow Cytometric Immunophenotyping" (see Calevetti et. al., 1993, Cytometry 14:702-714). Parametric (paired t test) and non-parametric (Wilcoxon) analyses were performed comparing levels of immune subset cell counts between dosing groups. Individual daclizumab exposure characteristics (results of *post hoc* analysis) from a subset of subjects, including steady state trough (C_{ss,min}), and AUCₛₛ, were used, separately, as predictors to model changes from baseline level in individual immune subsets or changes over time (calculated area under the change from baseline-time curve (AUC)). Relationship between changes from baseline level of putative activated T cell subsets and total new or enlarged Gd-CEL between Weeks 8 and 24 was also evaluated using linear correlation, negative binomial correlation, ANOVA or Kruskal-Wallis Tests statistical analysis approaches.

Analysis of daclizumab treatments at a dose of 2 mg/kg SC every 2 weeks, added to background treatment with interferon-beta produced statistically and clinically meaningful reductions in MS lesion activity (see FIG. 1) when compared to remaining on interferon-beta alone (and adding placebo).

Rapid reductions in absolute activated T cells were observed among patients in both daclizumab dose groups, but not in placebo-treated patients (see FIG. 2). As shown in FIG. 2, decreases from baseline in levels of absolute HLA-DR⁺ activated CD4⁺ T cells were observed for both dosing groups. Statistically significant reductions (p<0.05) in comparison to placebo are indicated (* DAC low dose and ** DAC high dose). Absolute levels of HLA-DR+ activated CD4+ T cells returned to approximate baseline levels at the time of CD25 de-saturation between days 196 to 280. At the last sampling at the end of the treatment period (week 22), mean levels in HLA-DR⁺CD4⁺ T cells were significantly lower in both DAC dose groups compared with placebo (DAC low dose vs. placebo: 22.5 vs. 37.4 cells/mcL, P=0.006; DAC high dose vs. placebo: 23.1 vs. 37.4 cells/mcL, P=0.009). Reductions in HLA-DR⁺CD4⁺ T cells reversed following discontinuation of DAC treatment.

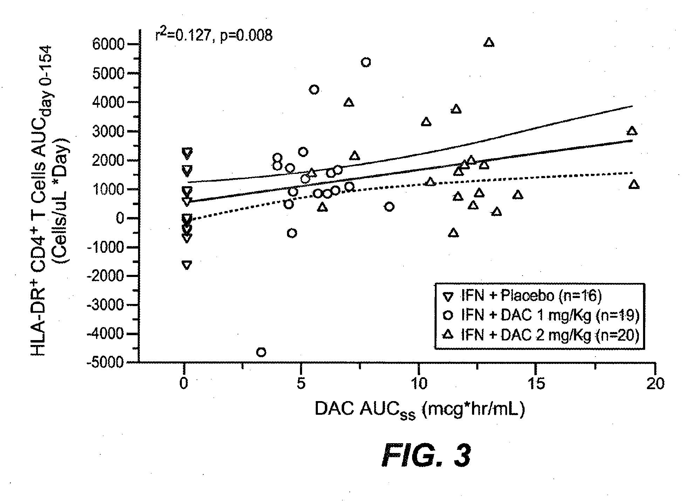

As shown in FIG. 3, a statistically significant (p=0.008) positive linear relationship was observed between individual DAC exposure at steady state and subject's reductions from baseline in levels of absolute HLA-DR⁺CD4⁺ T cell counts over time (within the dosing period), when each factor was expressed in terms of area under the curve (AUC) and when a reduction in HLA-DR⁺CD4⁺ T cell counts was expressed as a positive value (i.e. when a decrease in HLA-DR⁺CD4⁺ T cell counts from baseline was represented as a positive value). Thus, a significant positive relationship was demonstrated between daclizumab exposure and reduction in HLA-DR⁺CD4⁺ T cell counts (AUC) over time.

As shown in FIGS. 2 and 3, treatment of MS patients with daclizumab was associated with reductions in HLA-DR⁺CD4⁺ T cells; these reductions were rapid during the daclizumab treatment phase for both dosing groups and reversed after treatment with daclizumab was ended (see FIG. 2). A statistically significant, dose-dependent, positive relationship was observed between daclizumab exposure (AUCₛₛ) and reductions in HLA-DR⁺CD4⁺ T cell counts (AUC) over time when a reduction in HLA-DR⁺CD4⁺ T cell counts was expressed as a positive value (i.e. when a decrease in HLA-DR⁺CD4⁺ T cell counts from baseline was represented as a positive value) (see FIG. 3). The observed exposure-dependent effect of daclizumab in reducing activated T cell numbers during the treatment phase is consistent with the exposure-dependent clinical response. Taken together, these observations support the use of monitoring HLA-DR⁺CD4⁺ T cell counts as a biomarker of daclizumab antibody treatment in MS.

While various specific embodiments have been illustrated and described, it will be appreciated that various changes can be made without departing from scope of the invention(s).

## Claims

1. A method of monitoring the efficacy of an anti-IL-2R antibody in a patient diagnosed with multiple sclerosis, comprising determining the level of HLA-DR+CD4+ T cells, wherein a decrease in the level of HLA-DR+CD4+ T cells in the patient after exposure to the anti-IL-2R antibody indicates that the anti-IL-2R antibody is effective in ameliorating at least one symptom of multiple sclerosis in the treated patient.

2. The method according to claim 1, wherein the level of HLA-DR+CD4+ T cells is determined in blood samples from a patient prior to and subsequent to administering the anti-IL-2R antibody to said patient, wherein a decrease in HLA-DR+CD4+ T cells subsequent to said treatment indicates that the anti-IL-2R antibody is effective in ameliorating at least one symptom of multiple sclerosis in the treated patient.

3. A method according to claim 1 wherein the method comprises comparing the number of HLA-DR+CD4+ T cells in a blood sample obtained from the patient to an untreated reference, wherein a decrease in the number of HLA-DR+CD4+ T cells in the blood sample compared to the untreated reference indicates that the treatment is effective for ameliorating at least one symptom of multiple sclerosis in the subject.

4. The method according to claim 1, 2, or 3, wherein the antibody that specifically binds the interleukin 2 receptor is a humanized antibody.

5. The method according to claim 1 2, 3, or 4, wherein the anti-IL-2R antibody specifically binds to the alpha subunit of the human high-affinity interleukin-2 receptor and inhibits IL-2 signaling.

6. The method of claim 5, wherein the anti-IL-2R antibody is a humanized antibody.

7. The method of claim 6, wherein the humanized antibody is daclizumab.

8. The method according to anyone of the preceding claims wherein ameliorating a symptom of multiple sclerosis comprises reducing the number of relapses in a given period, reducing the rate of increase of the subject's Expanded Disability Status Score, reducing the number of T1 gadolinium contrast-enhanced MRI lesions, or reducing the number of T2 MRI lesions.

9. The method of claim 7, wherein daclizumab is administered at a dose of about 0.5 to about 5 milligrams per kilogram, or at a dose of about 1 to about 2 milligrams per kilogram.

10. The method of claim 7, wherein daclizumab is administered intravenously, subcutaneously, intramuscularly, intranasally, or transdermally.

11. The method of claim 7, wherein daclizumab is administered at least biweekly, or at least monthly.

12. The method according to any one of the preceding claims, wherein the subject has a relapsing form of multiple sclerosis.

13. The method according to claim 12, wherein the subject has relapsing remitting, secondary progressive, progressive relapsing, or worsening relapsing multiple sclerosis.

14. A method according to claim 1, comprising determining the number of HLA-DR+CD4+ T cells in blood samples collected from said patient prior to and subsequent to administering the anti-IL-2R antibody to said patient, to determine if a change in the number of HLA-DR+CD4+ T cells has occurred in the patient following treatment with the anti-IL-2R antibody.

15. The method according to claim 14, in which the change in the number of HLA-DR+CD4+ T cells in the treated patient is reduced by at least 25%, at least 50% or at least 100%.

## Patentansprüche

1. Verfahren zum Überwachen der Wirksamkeit eines Anti-IL-2R-Antikörpers bei einem Patienten, bei dem multiple Sklerose diagnostiziert wurde, umfassend Bestimmen des Levels von HLA-DR+CD4+-T-Zellen, wobei eine Abnahme beim Level von HLA-DR+CD4+-T-Zellen im Patienten nach Exposition gegenüber dem Anti-IL-2R-Antikörper anzeigt, dass der Anti-IL-2R-Antikörper bei der Besserung wenigstens eines Symptoms von multipler Sklerose bei dem behandelten Patienten wirksam ist.

2. Verfahren gemäß Anspruch 1, wobei der Level von HLA-DR+CD4+-T-Zellen in Blutproben von einem Patienten vor und nach Verabreichen des Anti-IL-2R-Antikörpers an den Patienten bestimmt wird, wobei eine Abnahme bei den HLA-DR+CD4+-T-Zellen nach der Behandlung anzeigt, dass der Anti-IL-2R-Antikörper bei der Besserung wenigstens eines Symptoms von multipler Sklerose bei dem behandelten Patienten wirksam ist.

3. Verfahren gemäß Anspruch 1, wobei das Verfahren Vergleichen der Zahl von HLA-DR+CD4+-T-Zellen in einer Blutprobe, die von dem Patienten erhalten wurde, mit einer unbehandelten Referenz umfasst, wobei eine Abnahme bei der Anzahl von HLA-DR+CD4+-T-Zellen in der Blutprobe im Vergleich zu der unbehandelten Referenz anzeigt, das die Behandlung zur Besserung wenigstens eines Symptoms von multipler Sklerose bei dem Subjekt wirksam ist.

4. Verfahren gemäß Anspruch 1, 2 oder 3, wobei der Antikörper, der den Interleukin-2-Rezeptor spezifisch bindet, ein humanisierter Antikörper ist.

5. Verfahren gemäß Anspruch 1, 2, 3 oder 4, wobei der Anti-IL-2R-Antikörper spezifisch an die alpha-Untereinheit des humanen Hochaffinitäts-Interleukin-2-Rezeptors bindet und IL-2-Signalübertragung inhibiert.

6. Verfahren gemäß Anspruch 5, wobei der Anti-IL-2R-Antikörper ein humanisierter Antikörper ist.

7. Verfahren gemäß Anspruch 6, wobei der humanisierte Antikörper Daclizumab ist.

8. Verfahren gemäß einem der vorangehenden Ansprüche, wobei Bessern eines Symptoms von multipler Sklerose Verringern der Anzahl von Wiederauftreten der Krankheit in einem gegebenen Zeitraum, Verringern der Anstiegsrate des Expanded Disability Status Score des Patienten, Verringern der Anzahl von T1-Gadoliniumkontrast-verstärkten MRI-Läsionen oder Verringern der Anzahl von T2-MRI-Läsionen umfasst.

9. Verfahren gemäß Anspruch 7, wobei Daclizumab mit einer Dosis von etwa 0,5 bis etwa 5 Milligramm pro Kilogramm oder mit einer Dosis von etwa 1 bis etwa 2 Milligramm pro Kilogramm verabreicht wird.

10. Verfahren gemäß Anspruch 7, wobei Daclizumab intravenös, subkutan, intramuskulär, intranasal oder transdermal verabreicht wird.

11. Verfahren gemäß Anspruch 7, wobei Daclizumab einmal alle zwei Wochen oder wenigstens monatlich verabreicht wird.

12. Verfahren gemäß einem der vorangehenden Ansprüche, wobei das Subjekt eine wiederkehrende Form von multipler Sklerose hat.

13. Verfahren gemäß Anspruch 12, wobei das Subjekt wiederkehrende nachlassende, sekundäre progressive, progressive wiederkehrende oder sich verschlimmernde wiederkehrende multiple Sklerose hat.

14. Verfahren gemäß Anspruch 1, umfassend Bestimmen der Anzahl von HLA-DR+CD4+-T-Zellen in Blutproben, die von dem Patienten vor und nach Verabreichen des Anti-IL-2R-Antikörpers an den Patienten gesammelt wurden, um zu bestimmen, ob eine Änderung in der Anzahl von HL-DR+CD4+-T-Zellen bei dem Patienten nach Behandlung mit dem Anti-IL-2R-Antikörper aufgetreten ist.

15. Verfahren gemäß Anspruch 14, wobei die Änderung in der Anzahl von HLA-DR+CD4+-T-Zellen bei dem behandelten Patienten eine Verringerung um wenigstens 25%, wenigstens 50% oder wenigstens 100% ist.

## Revendications

1. Procédé permettant de vérifier l'efficacité d'un anticorps anti-IL-2R chez un patient chez lequel a été diagnostiquée une sclérose en plaques, lequel procédé comporte le fait de déterminer le niveau des lymphocytes T CD4+ HLA-DR+, étant entendu qu'une baisse du niveau des lymphocytes T CD4+ HLA-DR+ chez le patient après exposition à l'anticorps anti-IL-2R indique que cet anticorps anti-IL-2R a bien pour effet d'améliorer au moins un symptôme de la sclérose en plaques chez le patient traité.

2. Procédé conforme à la revendication 1, dans lequel on détermine le niveau des lymphocytes T CD4+ HLA-DR+ dans des échantillons de sang prélevés chez un patient avant d'administrer l'anticorps anti-IL-2R audit patient et après le lui avoir administré, étant entendu qu'une diminution des lymphocytes T CD4+ HLA-DR+ à la suite de ce traitement indique que cet anticorps anti-IL-2R a bien pour effet d'améliorer au moins un symptôme de la sclérose en plaques chez le patient traité.

3. Procédé conforme à la revendication 1, lequel procédé comporte le fait de comparer le nombre de lymphocytes T CD4+ HLA-DR+ dans un échantillon de sang prélevé chez le patient à une référence non traitée, étant entendu que si le nombre des lymphocytes T CD4+ HLA-DR+ dans l'échantillon de sang est moindre que dans la référence non traitée, cela indique que ce traitement a bien pour effet d'améliorer au moins un symptôme de la sclérose en plaques chez le sujet.

4. Procédé conforme à la revendication 1, 2 ou 3, dans lequel l'anticorps qui se lie spécifiquement au récepteur d'interleukine 2 est un anticorps humanisé.

5. Procédé conforme à la revendication 1, 2, 3 ou 4, dans lequel l'anticorps anti-IL-2R se lie spécifiquement à la sous-unité alpha du récepteur humain de haute affinité pour l'interleukine 2 et inhibe la signalisation de l'interleukine 2.

6. Procédé conforme à la revendication 5, dans lequel l'anticorps anti-IL-2R est un anticorps humanisé.

7. Procédé conforme à la revendication 6, dans lequel l'anticorps humanisé est du daclizumab.

8. Procédé conforme à l'une des revendications précédentes, dans lequel l'amélioration d'un symptôme de la sclérose en plaques comprend la diminution du nombre de récidives au cours d'un laps de temps donné, le ralentissement de la hausse de la cote du sujet sur l'échelle élaborée d'incapacité (EDSS), la réduction du nombre de lésions détectées en IRM en T1 à contraste renforcé au gadolinium, ou la réduction du nombre de lésions détectées en IRM en T2.

9. Procédé conforme à la revendication 7, dans lequel le daclizumab est administré en une dose d'environ 0,5 à environ 5 milligrammes par kilogramme, ou en une dose d'environ 1 à environ 2 milligrammes par kilogramme.

10. Procédé conforme à la revendication 7, dans lequel le daclizumab est administré par voie intraveineuse, sous-cutanée, intramusculaire, intranasale ou transdermique.

11. Procédé conforme à la revendication 7, dans lequel le daclizumab est administré au moins une fois toutes les deux semaines ou au moins une fois par mois.

12. Procédé conforme à l'une des revendications précédentes, dans lequel le sujet présente une forme récurrente de la sclérose en plaques.

13. Procédé conforme à la revendication 12, dans lequel le sujet présente une sclérose en plaques récurrente-rémittente, secondairement progressive, progressive-récurrente, ou récurrente aggravée.

14. Procédé conforme à la revendication 1, comportant le fait de déterminer le nombre de lymphocytes T CD4+ HLA-DR+ dans des échantillons de sang prélevés chez ledit patient avant d'administrer l'anticorps anti-IL-2R audit patient et après le lui avoir administré, afin de déterminer s'il s'est produit une variation du nombre de lymphocytes T CD4+ HLA-DR+ chez le patient à la suite du traitement avec cet anticorps anti-IL-2R.

15. Procédé conforme à la revendication 14, dans lequel la variation du nombre de lymphocytes T CD4+ HLA-DR+ chez le patient traité est une réduction d'au moins 25 %, ou d'au moins 50 %, ou d'au moins 100 %.
